# EUROPEAN PATENT SPECIFICATION

(11) **EP 1 734 907 B1**
(45) Date of publication and mention of the grant of the patent: **17.08.2011**
(21) Application number: 05729648.5
(22) Date of filing: 24.03.2005
(51) Int. Cl.: A61F 2/44, A61F 2/46

(54) **POROUS IMPLANT FOR SPINAL DISC NUCLEUS REPLACEMENT**
PORÖSES IMPLANTAT FÜR DEN BANDSCHEIBENNUKLEUS-ERSATZ
IMPLANT POREUX DE REMPLACEMENT DU NOYAU DU DISQUE SPINAL

(30) Priority: 26.03.2004 GB 0406851; 05.04.2004 GB 0407717
(43) Date of publication of application: 27.12.2006
(73) Proprietor: NuVasive, Inc., San Diego CA 92121 (US)
(72) Inventor: MCLEOD, Alan, Rory, Mor, Pearsalls Limited, Taunton, Somerset TA1 1RY (GB); REAH, Christopher, Pearsalls Limited, Taunton, Somerset TA1 1RY (GB)
(74) Representative: Merrifield, Sarah Elizabeth
(86) International application number: PCT/GB2005/001179
(87) International publication number: WO 2005/092248

(56) References cited:
- EP-A- 0 621 020
- WO-A-92/10982
- US-A- 6 093 205
- US-B1- 6 416 776

## Description

This invention concerns improvements in and relating to implants, for instance spinal implants and more particularly, but not exclusively in relation to full or partial replacement of the nucleus pulposus.

A number of surgical techniques are known with a view to addressing problems with intervertebral discs. Such techniques include the partial or complete removal of the nucleus pulposus or remains thereof. In other cases the entire disc is removed. The subsequent treatment varies. In some cases the two vertebrae are fused together, for instance by tight packing of bone chips between the two. In some other case, an implant of some design is inserted into the space vacated. The implant may seek to provide some of the function of the disc or merely maintain the spacing of the vertebrae. Such treatments may be needed because of damage and/or disease.

In cases where only part of the nucleus has been lost or is to be replaced, generally, one or more incisions are made in the annulus of the disc. The necessary part of the nucleus is then removed through the opening created by the incisions. The implant itself is then inserted. The implant is inserted whole or may be inflated after insertion by introducing hydrogel or the like. Once installed, the incisions are closed and sutured. The sutures are applied direct to the annulus itself with the sutures passing through the annulus on one side of the incision, bridging the incision and then passing through the annulus on the other side.

The success of such a procedure depends upon the long term ability of the implant to provided the desired spacing between vertebrae and the extent to which natural function of the disc is maintained.

WO 92/10982 discloses a spinal implant including a porous component, such as a polymer membrane, and one or more filling elements, such as hydrogel beads, provided within the porous component.

The present invention has amongst its aims potentially to provide long term maintenance of the spacing between vertebrae. The present invention has amongst its aims potentially to provide better tissue ingrowth into and/or around the implant. The present invention has amongst its aims to maintain as far as possible natural function for the spine.

The present invention provides a spinal implant as set out in claim 1.

According to the invention we provide a spinal implant, the implant including a porous component and one or more filling elements provided within the porous component. One or more materials in the porous component is bioabsorbable.

The bio-absorbable material restrains the porous component in a first state, the bio-absorption of the material allowing the porous component to assume a second state.

The implant may be a partial nucleus pulposus replacement. The implant may be a total nucleus replacement. Preferably the implant maintains the separation of the vertebrae which it is provided between. The implant may mimic the characteristics of a naturally occurring nucleus. Preferably the implant provides some or all of the resistance to compressive loads provided by a natural nucleus. The implant may be inserted anteriorly or posteriorly. The implant may be provided within a natural annulus and / or an artificial annulus.

The porous component may be a bag or other form of container. The porous component may have an opening to permit the insertion of the one or more filling elements. Preferably the opening is closable, for instance by one or more of folding, stitching, suturing, gluing, stapling or the like.

The porous container may be made of fabric, particularly a woven fabric. The fabric may be one or more of flat woven or circular woven, knitted, braided, embroidered or combinations thereof.

The fabric may include and/or be formed from one or more fibre materials. The fabric may include and/or be formed of one or more of polyester, polypropylene, polyethylene, glass fibre, glass, polyaramide, metal, copolymers, polylactic acid, polyglycolic acid, biodegradable materials, silk, cellulose or polycaprolactone.

Preferably the pores in the porous component have at least one cross-sectional dimension that is less than the smallest cross-sectional dimension of the filling elements. Preferably the cross-sectional area of the pores is less than the minimum cross-sectional area of the filling elements. Preferably the filling elements cannot pass through the pores of the porous component.

The porous component may entirely surround the filling elements and/or encapsulate the filling elements. One or more apertures or gaps may be provided in the porous component, ideally to provide or assist fluid communication through the porous component or to assist it. Preferably a large number of apertures or gaps are provided in the material from which the porous component is formed, for instance a woven fabric. The apertures or gaps occurring in the porous component may be due to the manner of manufacture of the material from which it is formed or may be supplemented with further apertures or gaps. The supplementation may be provided by degradation and/or absorption of one or more materials forming the inner component. Where apertures or gaps are provided, preferably they have at least one cross-sectional dimension that is less than the smallest cross-sectional dimension of the filling elements. Preferably the cross-sectional area of the gaps or apertures is less than the minimum cross-sectional area of the filling elements. Preferably the filling elements cannot pass through the gaps or apertures of the porous component.

The porous component may be configured and/or formed of and / or provided with one or more materials intended to promote tissue growth, particularly tissue ingrowth through the porous component and/or between the porous component and one or more of the filling elements and/or between two or more of the filling elements. Tissue growth may be promoted by the material type, for instance polyester. Tissue growth may be promoted by the configuration, particularly the size and/or number of pores and/or gaps and/or apertures in the porous component. Tissue growth may be promoted by a chemical, for instance a pharmaceutical, provided as part of the porous component or associated therewith.

The bio-absorbable material may be used to decrease the amount of porous component present and/or positions at which the porous component is present and/or density at which the porous component is present overtime. The second state of the porous component may provide a greater internal volume for the porous component and/or greater porosity for the porous component and/or reduction in mass of the porous component and/or provide more space for tissue ingrowth.

Bio-absorbable material may be incorporate in the porous component by providing areas of bio-absorbable material and/or one or more fibres of bio-absorbable material. The porous component may be entirely bio-absorbable or only partially. Different materials having different rates of bio-absorption may be used for different areas and/or different fibres within the porous component. Slow, moderate and fast bio-absorption materials may be used.

The one or more filling elements may be fibrous and/or formed of single filaments.

One or more of the one or more filling elements may include and/or be formed from one or more fibre materials. One or more of the one or more filling elements may include and/or be formed of one or more of polyester, polypropylene, polyethylene, glass fibre, glass, polyaramide, metal, copolymers, polylactic acid, polyglycolic acid, biodegradable materials, silk, cellulose or polycaprolactone.

Preferably one or more filling elements that are porous and/or define voids within themselves and/or between parts of a filling element are provided. The pores and/or voids and/or apertures and/or gaps provided in or by the filling elements ideally provide fluid communication through the filling elements and/or there between. Preferably a large number of pores and/or voids and/or apertures and/or gaps are provided in the material from which filling elements are formed. Preferably a large number of pores and / or voids and / or apertures and / or gaps are provided by one or more of the filling elements. Preferably a large number of pores and / or voids and / or apertures and / or gaps are provided within one or more of the filling elements by virtue of their structure.

One or more filling elements may be formed of unconstrained fibres. One or more filling elements may be formed of unbraided fibres. One or more filling elements of felt or felt-like material may be provided. One or more filling elements with interlaced fibres may be provided. One or more filling elements may be provided with aligned fibres. One or more filling elements may be provided with one or more groups of aligned fibres and / or one or more non-aligned fibres and / or one or more groups of fibres on different alignments to the first. One or more filling elements with non-linear fibres may be provided. One or more filling elements with wavy and / or curved and / or zig zag fibres may be provided. One or more filling elements with fibres which act to space each other from one another may be provided. One or more filling elements with primary fibres having a first alignment and secondary fibres on a different alignment, which serve to space the primary fibres from one another may be provided. One or more filling elements of cotton wool or like material may be provided.

One or more filling elements with fibres of two or more different cross sections may be provided. The fibres of different cross sections may be linear and / or non-linear.

One or more filling elements with fibres provided in a first direction may be provided, with one or more restraining fibres or material. The restraining fibres and / or material may surround and / or enclose and / or be wrapped around and / or contact a plurality of fibres. The restraining fibre or material may be provided as a band. The restraining fibres of material may be provided at the ends of the filling elements and / or at intermediate locations thereon.

One or more filling elements may be provided with peripheral fibres or material provided around the filling element. The peripheral fibres or material may be wrapped around the filling elements in a spiral manner and / or criss-cross manner. The fibres or material may be provided in an anti-clockwise and / or clockwise manner. A fishnet of fibres may be provided around one or more filling elements.

One or more filling elements may be provided with pieces provided therein. The pieces may be intermixed with one or more fibres. The pieces may be spheres, beads, blocks or the like. The pieces may be integral with the fibres and / or connected thereto and / or free to move relative to the fibres. Preferably fibres are wrapped and / or extend around at least part of the periphery of the beads, ideally in a variety of directions. The pieces may be linked together by a fibre or filament, particularly in the case of the series of spheres. The spheres may be surrounded by a mass of braided fibres. The masses of braided fibres may be linked by one or more fibres or filaments. Preferably the masses of fibres surround the spheres.

A single layer of filling elements may be provided within the porous component. Multiple layers of filling elements may be provided within the porous component. One or more intermingled filling elements may be provided within the porous component. The filling elements may be of linear configuration and / or curved and / or wavy. One or more spiral filling elements may be provided. One or more filling elements of substantially circular cross-section may be provided. One or more filling elements with one or more flat surfaces may be provided. One or more filling elements of generally square and / or pentagonal and / or hexagonal and / or octagonal cross-section may be provided.

The pores and/or voids and/or apertures and/or gaps occurring in the filling elements and/or there between may be due to the manner of manufacture of the material from which it is formed or may be supplemented with further pores and/or voids and/or apertures or gaps. The supplementation may be provided by degradation and/or absorption of one or more materials forming the filling elements.

The one or more filling elements may be configured and/or formed of one or more materials intended to promote tissue growth, particularly tissue ingrowth through one or more filling elements and/or between the porous component and one or more filling elements and/or between two or more of filling elements. Tissue growth may be promoted by the material type, for instance polyester, included in one or more filling elements. Tissue growth may be promoted by the configuration, particularly the size and/or number of pores and/or gaps and/or apertures in one or more filling elements.

One or more materials used in one or more of the filling elements may be bio-absorbable. The bio-absorbable material may be used to decrease the amount of one or more filling elements present and/or positions at which one or more filling elements is present and/or density at which one or more filling elements is present overtime. The bio-absorbable material may restrain one or more of the filling elements, or a part thereof, in a first state, the bio-absorption of the material allowing one or more filling elements, or a part thereof, to assume a second state. The second state may provide a greater internal volume for one or more filling elements and/or greater porosity for one or more filling elements and/or reduction in mass of one or more filling elements and/or provide more space for tissue ingrowth.

Bio-absorbable material may be incorporate in one of more filling elements by providing areas of bio-absorbable material and/or some fibres of bio-absorbable material. One or more of the one or more filling elements may be entirely bio-absorbable or only partially. Different materials having different rates of bio-absorption may be used for different areas and/or different fibres within one or more filling elements. Slow, moderate and fast bio-absorption materials may be used.

The invention may include any of the features, options or possibilities set out elsewhere in this document.

Also described is a surgical technique in which, at least part of the spinal disc is removed and an implant is provided, the implant having a porous component and one or more filling elements provided within the porous component..

A part or the whole of a nucleus pulposus may be replaced. The implant may be inserted anteriorly and / or posteriorly.

Preferably the porous component is inserted through the same incision as is used to remove the nucleus material. Preferably the incision is only as large as needed for the nucleus material removal stage. The porous component may be folded and / or compressed for insertion into the intervertebral disc space. Preferably the one or more filling elements are absent from the porous component during insertion into the intervertebral disc space.

Preferably the one or more filling elements are introduced into the porous component within the intervertebral disc space. The one or more filling elements may be deployed from an applicator, for instance by extrusion therefrom.

Preferably the one or more filling elements are provided through the incision used to remove the nucleus material. Preferably the incision used for introducing the one or more filling elements is no larger than the incision necessary for the removal of the nucleus material.

Preferably the technique includes a first time in which the implant provides one or more characteristics of a naturally occurring disc by virtue of a non-biological mechanism, and a second time at which the implant provides one or more characteristics of naturally occurring disc by a combination of a non-biological mechanism and biological mechanism. Ideally, the biological mechanism is tissue in-growth. The technique may include a third time with substantially all of the one or more characteristics of a naturally occurring disc are provided by a biological mechanism. Preferably the transition from the mechanism at the first time to the second time and / or third time is due to bio-absorption of one or more of the materials forming the implant and particularly forming one or more filling elements thereon.

Various embodiments of the invention will now be described, by way of example only, and with reference to the accompanying drawings in which:-
Figure 1 shows a perspective view of a disc featuring part of a device according to an embodiment of the present invention;
Figure 2 shows the view of Figure 1 with the device near completion;
Figure 3 shows the dispensing of one embodiment of the filling using one embodiment of an applicator;
Figures 4a to 4c show other embodiments of fillings;
Figure 5 shows a further embodiment of a filing in perspective view;
Figures 6a and 6b shows still further embodiments of filings in perspective view;
Figure 7 shows yet another embodiment of a filling;
Figure 8 shows an embodiment of the invention including beads;
Figure 9 shows a further bead incorporating embodiment of the invention; and
Figure 10a to 10c show different stages in the life of a device according to the invention, from initial point of deployment, through an intermediate time to a much later time after deployment.

Each of the intervertebral discs within a spine function as a spacer, as a shock absorber, and to allow motion between adjacent vertebrae. The height of the disc maintains the separation distance between the vertebral bodies. There are three functions that the intervertebral disc performs:-
- Proper spacing - Allows the intervertebral foramen to maintain its height, allowing the segmental nerve roots, room to exit each spinal level without compression.
- Shock absorption - Not only allows the spine to compress and rebound when the spine is axially loaded (during such activities as jumping and running) but also to resist the downward pull of gravity on the head and trunk during prolonged sitting and standing.
- Elasticity (of the disc) Allows motion coupling, so that the segment may flex, rotate, and laterally bend all at the same time during a particular activity. This would be impossible if each spinal segment were locked into a single axis of motion.

The intervertebral disc consists of four distinct parts. These are the nucleus pulposus, annulus fibrosus and two end plates. It should be noted that although these four sections are very much distinct in their own right the boundaries between then are not as distinct. Most investigators tend to ignore the end plates and dismiss them as merely as the barrier between the vertebrae and the parts of the disc which allow motion of the spine. However, the end plates are important in completing the structure of the disc and creating some of the boundary conditions that define the behavior of the disc.

From around the 20th year of a persons life, the discs become completely avascular, although they show high metabolic turnover. The water content of the discs will decrease the older the person gets.

### End Plates

The end plates are composed of hyaline cartilage. This is basically a "hydrated Proteoglycan gel, reinforced by Collagen Fibrils" - Ghosh; The Biology of the Intervertebral Disc. CRC Press, ISBN 084936711523. As stated, the boundary between the annulus and end Plate is not a distinct one, under a microscope the two parts merge together, with a region which is neither one tissue nor the other.

### Annulus

The annulus is the outer ring of the disc. A strong, laminated structure of opposed layers of Collagen fibres. An annulus typically comprises around 12 laminae, with 6 provided in each direction of fibre travel. The layers are at an angle of approximately 30° on every other layer, with 30° in the opposite direction on the remaining layers. The functions the annulus performs determine the need for this type of structure. No matter which direction the vertebrae moves, there will always be some fibres in tension and some in compression. Thus, the annulus will always be acting using some fibres to stretch (they will resist stretch like an elastic band) and pull the spine back into the correct posture.

The annulus has overlapping, radial bands, not unlike the plies of a radial tyre, and this allows torsional stresses to be distributed through the annulus under normal loading, without rupture. One study suggested that the posterior part of the annulus is the weaker side, so more susceptible to damage - Tsuji; Structural variation of the annulus fibrosis. Spine 18 pp204-210, 1993. The annulus is the strongest part of the disc.

### Nucleus

The nucleus at the centre of the disc, is a highly hydrated gel of Proteoglycans. In children and young adults, the water content can account for up to 80% of its weight - Ghosh. This gel material is a very thick fluid that is dense enough to be able to be torn. It serves the twin purposes of both direct load bearing and, by being fluid in nature, being able to change shape under loading to distribute the load to the annulus. The nucleus may only bear half the load of the FSU (functional spinal unit) with the annulus carrying the rest-Finneson; Low back pain. ISBN 0-397-50493-4, 1992. It is this shared loading that allows the disc to continue to operate even after the nucleus has been damaged.

Degeneration and/or herniation and/or damage to a disc can occur during a patient's life.

Degenerative disc disease (DDD) is the process of a disc losing some of its function, due to a degenerative process, and is a very common and natural occurrence. At birth the disc is comprised of about 80% water. As ageing occurs, the water content decreases and the disc becomes less of a shock absorber, the proteins within the disc space also alter their composition.

The relationship between degeneration and pain is not a clear one. Theories to explain why some degenerative discs are painful include:
Injury : A tear in the annulus may release nucleus material, which is known to be inflammatory.

Nerve Ingrowth into Discs : Some people seem to have nerve endings that penetrate more deeply into the outer annulus, than others, and this is thought to make the disc susceptible to becoming a pain generator.

Loss of Height : A degenerative disc may lose height as the water content lowers. This may cause the disc to bulge outwards - pressurising the nerve roots and thus causing pain. In addition, this loss in height will have other effects that can also be pain generating:
The disc biomechanics will alter. Normally the nucleus pressurizes the annulus forcing the fibres into tension. However in these cases the nucleus will lose this ability and the annulus itself will be forced to carry the compressive load at that level in the spine. This will increase the stress in the annulus.
The load distribution through the disc will be affected by this. When the uniform distribution becomes more haphazard the load will not be carried in an even manner throughout the disc.
Alteration in the disc biomechanics will affect both the patient's range of motion and alter the position of the instantaneous axis of rotation in normal movements.
The result of these factors will usually mean increased loading on the facet joints that may in turn start to degenerate and become symptomatic.

What ever the reason behind the degeneration causing the pain, treatment to improve the position and the patient's life is important. The treatment options are discussed in more detail below.

A herniated disc is similar to a prolapsed one, in that there is a bulge in the disc itself. However, the disc will not have collapsed in the same way. The injury is thought to be through a combination of a degenerative process and mechanical loading. The stages of disc herniation - Ibrahim; ; Colorado spine institute; http://www.coloradospineinstitute.com *2004;* are disc degeneration, perhaps due to chemical changes associated with aging cause the disc to weaken; formation of a bulge due to this localised failure of the annulus; progression of the condition can cause the nucleus to protrude out as a herniation; the bulge will press against the nerves in the spinal canal and cause pain that the body sees as coming from the legs; further progression results in extrusion as the gel-like nucleus pulposus breaks through the annulus fibrosis, but remains within the disc; further progression may result in the nucleus pulposus breaking through the annulus fibrosus and lying outside the disc in the spinal canal, a sequestered disc.

Whilst most patients with a herniation will improve without surgery in some case surgery is necessary. If surgery is required then usually the treatment will be to remove part, or all of the herniated disc, such that the nerve roots are no longer impinged.

### Surgical treatment of Degenerative and Herniated Discs

When a disc that is showing signs of degeneration or herniation or disease or damage become painful a surgeon may often operate. Treatments that may be conducted include:
1. Partial discectomy - removal of local annular material to the site of a herniation.
2. Partial nucleotomy - removal of local nucleus material close to the site of the herniation.
3. Discectomy and fusion - removal of the entire disc and fusion of the disc space, used in more serious cases.
4. Other treatments, such as a disc replacement or nucleus replacement - these are new treatments used as an alternative to fusion.

The present invention is intended to be particularly useful as part of the following treatments:
- *Nucleotomy* to replace the lost or removed nucleus material by inserting the implant to provide a nucleus material equivalent alongside the remaining nucleus material;
- *Artificial Disc Replacement* to restore a functional nucleus by inserting the implant in conjunction with an annulus replacement;
- *Artificial Nucleus Replacement* to restore a functional nucleus by inserting the implant.

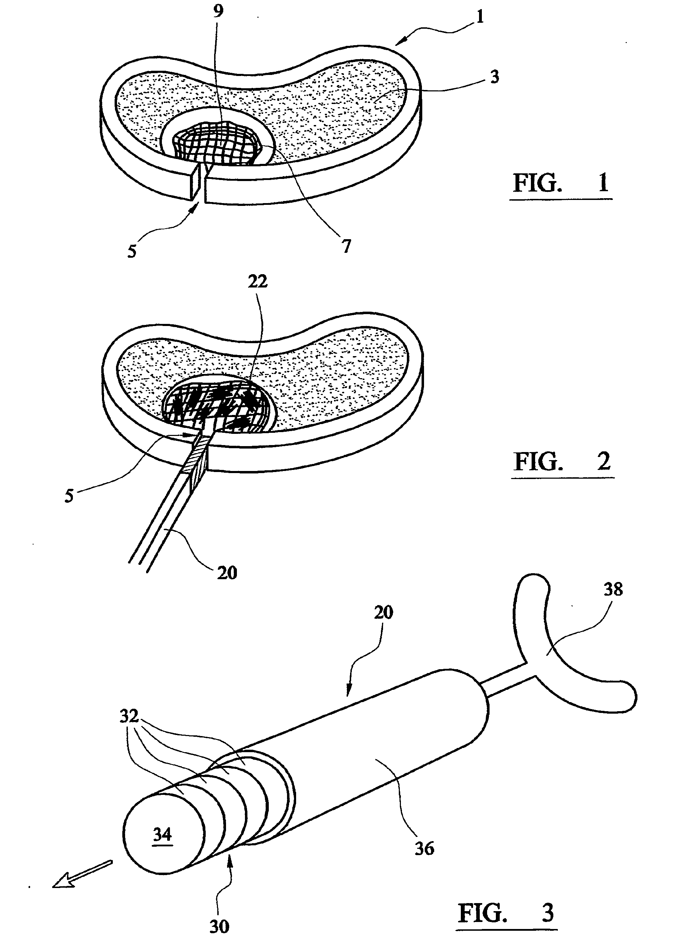

With reference to Figure 1, an intervertebral disc 1 is shown with part of the nucleus 3 removed through an incision 5. Following removal of the material, the first part of the implant has been inserted. The first part is a fabric bag 7 with an opening 9. The bag 7 is empty and hence easily reduced to a small size at this stage so as to allow easy insertion through the incision 5. The incision 5 is of the smallest size necessary to remove the nucleus material. This comes with prior art systems where the incision 5 needed for the nucleus removal needed to be enlarged to allow enough room to deploy the implant. The opening 9 into the bag is kept close to the incision 5.

The bag 7 is formed in such away as to offer the necessary strength and structural properties to constrain the filling it is to receive, but does so whilst being open to the passage of fluid through it, both into and out of its inside. The significance of this will be described in greater deal below.

In Figure 2, the next stage of the implants formation is shown. Using an applicator 20, the second part of the implant, the filling 22 is pushed into the bag 7 through the opening 9. The filling 22 is of relatively small cross-section and so does not necessitate any enlargement of the incision 5 either. Sufficient filling 22 is introduced into the bag 7 to give it the desired properties discussed in more detail below. As can be seen, however, the filling 22 causes the bag 7 to generally assume the profile of the space in the nucleus 3.

The filing 22 is made of one or more material which encourage tissue growth, such as polyester fibre.

Such a bag can be provided together with (as in used alongside but discrete from) or linked to or as an integral part of the type of device disclosed in WO 2005/092211.

An implant according to the present invention is suitable when a procedure such as a nucleotomy has been conducted as the disc will have lost material from the nucleus. This may cause a loss in nucleus function and/or a loss in disc height. The implant thus provides a partial artificial and so provides treatment in these cases.

An important part of the present invention is the filling 22 used and structure of the bag 7.

In disc / nucleus replacement procedures, the prior art approach has been to provide a non-biological mechanism for mimicking the disc's natural function throughout the life of the device. As far as practically possible the device has been isolated from its biological surrounds. The present invention aims to provide a phased transition from a solution based on a non-biological mechanism to a combination of biological and non-biological mechanisms and potentially even on to a predominantly or even exclusively biological mechanism.

This aim can be achieved by careful design of the filling 22 and bag 7 to facilitate rather than resist tissue ingrowth.

When exposed to alien materials which cannot be expelled or broken down, the bodies reaction is to try and isolate the material. Tissue thus grows around the material.

In the past, the continuous nature of the implant has meant that the tissue has grown only around the outside of the implant. In the case of inflatable balloons, this is because the outer which constrains the inflation, by its very nature, also prevents tissue growth inside. Similarly metal devices prevent tissue ingrowth because of the material they are made from. Other implants have used an outer which is continuous in nature and so only a surface layer of tissue around the very outside may have developed. Either because to the nature of the implant or because of active steps taken, no tissue ingrowth within the implant occurs. In some cases, steps to actively avoid tissue ingrowth have been taken, for instance to prevent the tissue interfering with the operation of the non-biological mechanics of the device.

The present invention takes a fundamentally different approach and actively seeks tissue ingrowth for the implant.

Firstly, the bag 7 is provided in such a way that there are significant openings/gaps between the fibres forming the bags. Fluids can thus readily pass through the bag 7 in either direction. As a result the outer of the implant facilitates tissue ingrowth through itself.

Secondly, and with reference to the Figure 2 embodiment, the filling 22 consists of groups of fibres collected together in an unconstrained, unbraided mass. The elongate nature of the fibres suits them to alignment within the applicator 20. Some alignment is retained within the bag 7, but generally the result is a filling 22 formed of an open mass of fibres.

Such a filling 22 of unconstrained and unbraided polyester filaments or fibres initially occupies a small volume in the nucleus. Following implantation, however, tissue ingrowth into the filling 22 occurs. The open nature of the mass of fibres and material of the fibres promotes this. With time, the tissue ingrowth tends to surround each fibre individually, as the tissue is able to reach each individually. Thus each individual fibre is alien material to be isolated by surrounding. If densely packed fibres are provided, the tissue growth is again restricted to the outside as the fibres are seen as an integral mass by the tissue. The open fibres of the present invention in effect act as a scaffold. As this growth progresses, it will cause the volume of the filling 22 and hence the bag 7 to swell to fill the available space in the nucleus.

The lack of restriction on the tissue ingrowth and the free access for fluids into and out of the bag 7 and filling 22 should mean that the tissue which grows is similar in composition and hence properties to the undisturbed nucleus material that surrounds it.

The swelling of the bag 7 should restore some of the disc height that has been lost as the disc failed.

In theory, during the earlier stages of degenerative disc disease, the idea of refilling the nucleus with scaffolding polyester fibre could act as a permanent treatment. At the very least, it would be expected to improve the patient's condition in the medium term delaying a more serious procedure. In the meantime, all normal treatment options would still be able to be used on the patient.

The applicator 20 is illustrated in more detail in Figure 3, in conjunction with a different form of filling 30, to the filling 22 used in the Figure 2 embodiment. In this case, rather than being a mass of fibres in an unconstrained form, the filling 30 is provided in the form of a number of discrete pads 32 of felt like material 34. Felt and similar materials used the natural interlacing of their fibres to form an open porous structure. This can be supplemented by needling to increase the interlacing and/or openness of the structure if desired.

The applicator 20 consists of a tube 36 which holds the pads 32. Under the control of the surgeon a plunger 38 is advanced in the tube 36 to push the pads 32 out into the bag 7 within the disc. Overtime, the pads 32 expand as tissue grows within and around them. Different applicator cross-sections can be used to deploy different fillings.

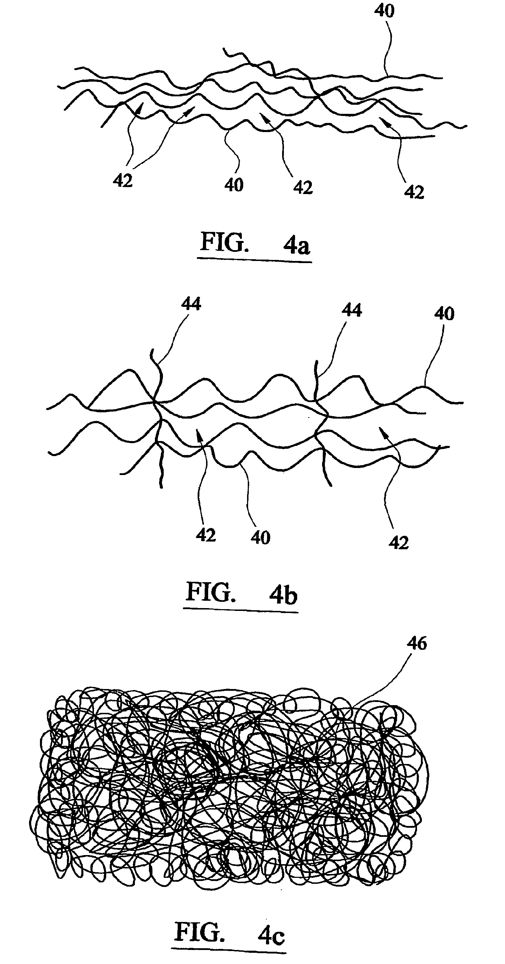

Figures 4a, 4b and 4c illustrate a number of alternative forms of filling 22 in unconstrained, unbraided form. Figure 4c shows a series of generally aligned fibres 40 which are non-linear in nature. The waves built into the fibres 40 serve to space individual fibres 40 from one another. The result is a mass of fibres 40 with substantial voids 42. Figure 4b shows a modification, in which a series of secondary fibres 44 are provided with a different orientation to the primary fibres 40. The difference in orientation resists pressures which would otherwise cause the voids 42 between the fibres to be reduced.

Figure 4c shows a mass of fibres 46 in a form more closely approaching that of a felt or cotton wool material. A very large number of different orientations are provided and thus serve to maintain the spacing against compression in a wide variety of directions.

The fibre could be provided from staple fibre, potentially subsequently chopped into short lengths. The fibre could be used as supplied or be modified before or after chopping, potentially to provide braiding or other restraining surround. It is possible to use fibres form of single filaments and/or filaments twisted together and/or braided together.

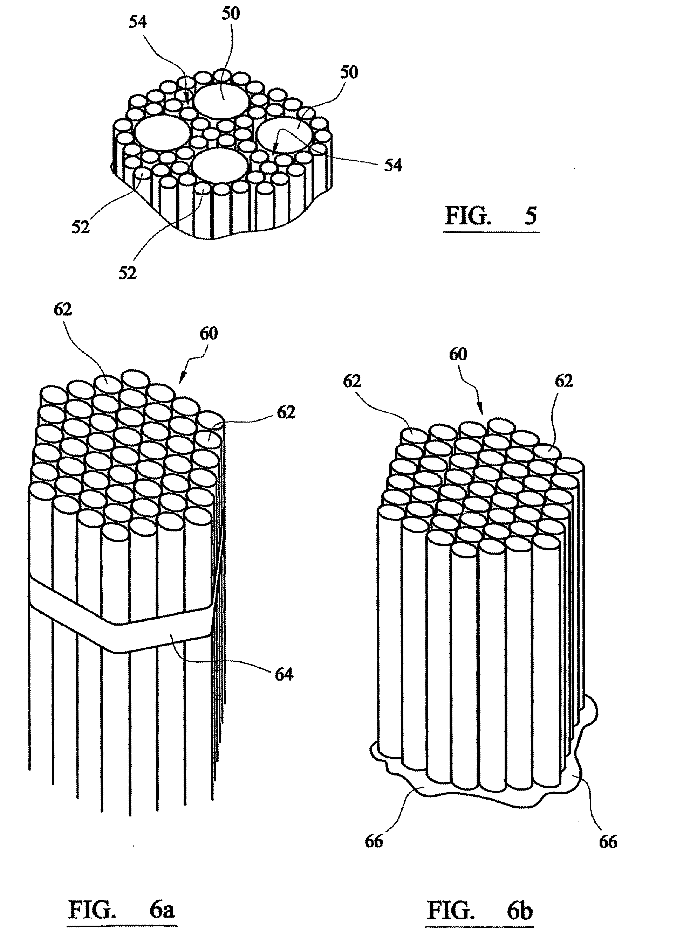

Figure 5 shows a further filling form in which primary fibres 50 of a large cross-section are mixed with secondary fibres 52 of a smaller cross-section. The differences in cross-section again help to maintain the voids 54 within the filling.

Figures 6a and 6b illustrate examples of a more structured filling 60. In the first case, Figure 6a, the majority of the fibres 62 are provided along a first alignment. To assist in keeping the alignment of the fibres 62 during and after deployment, a limited number of fibres 64 are wrapped around the fibres 62 to maintain them as bundles. The bundles are still open, however, and have significant voids. In the Figure 6b form, the fibre bundle is chopped by a hot blade and this melts part of the ends and joins them together upon cooling due to mass 66.

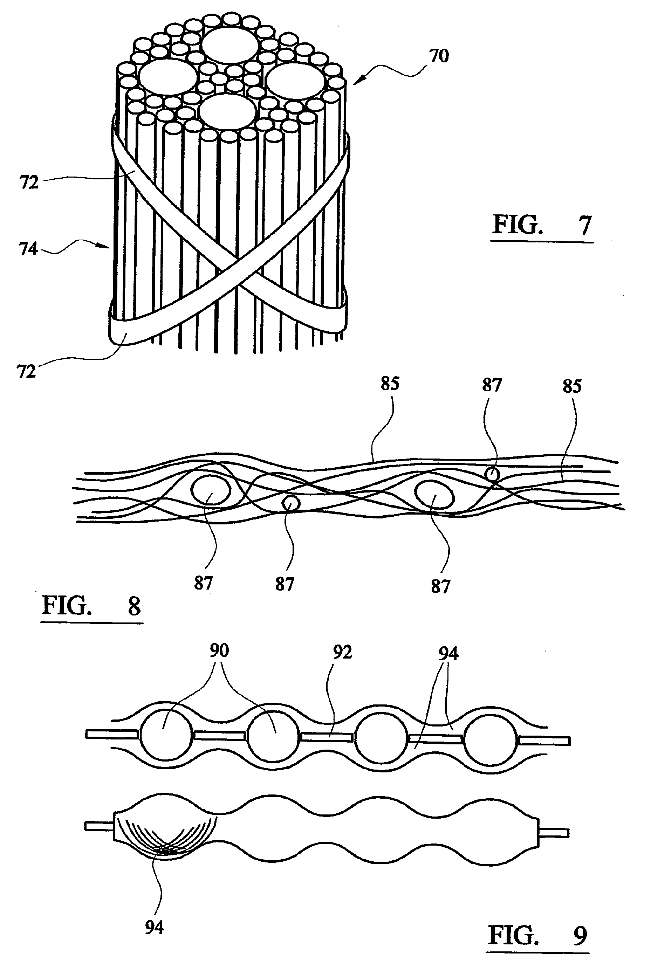

The Figure 7 embodiment is a still more structured embodiment of the filling 70. An outer layer of criss-cross fibres 72 is provided so as to maintain the inner fibres 74 in the desired position. The inner fibres 74 are a mixture of large 76 and small 78 fibres. By potentially providing the fibres 76, 78 on a number of slightly different alignments a more open structure with large voids is provided. The large gaps in the outer layer of criss-cross fibres 72 means that there is no interference with tissue ingrowth, but these fibres can be provided with a degree of stiffness to assist deployment and positioning of the filling 70 within the space in the bag which surrounds it. A series of lengths of such filling 70 can be used in a single bag to give the desired overall structure.

In Figure 8, the fibres 85 within a bundle are spaced and provided on a variety of alignments by the inclusion of a number of spherical beads 87.

Finally in Figure 9, a series of beads 90 are provided linked together by a fibre 92. The beads are each surrounded by a mass of fibres 94 braided on to form a mass. The braided mass 94 surrounds each of the beads 90 like a sleeve. Again the filling itself is open and promotes tissue growth.

In many of the above cases, the desired open structure is not only provided by individual groups of fibres, but also by the interaction between individual groups of fibres and the voids between them that they define.

In all of the above embodiments, and in the invention in general, the provision of an open structure can also be assisted by the careful use of different materials for different parts of the filling.

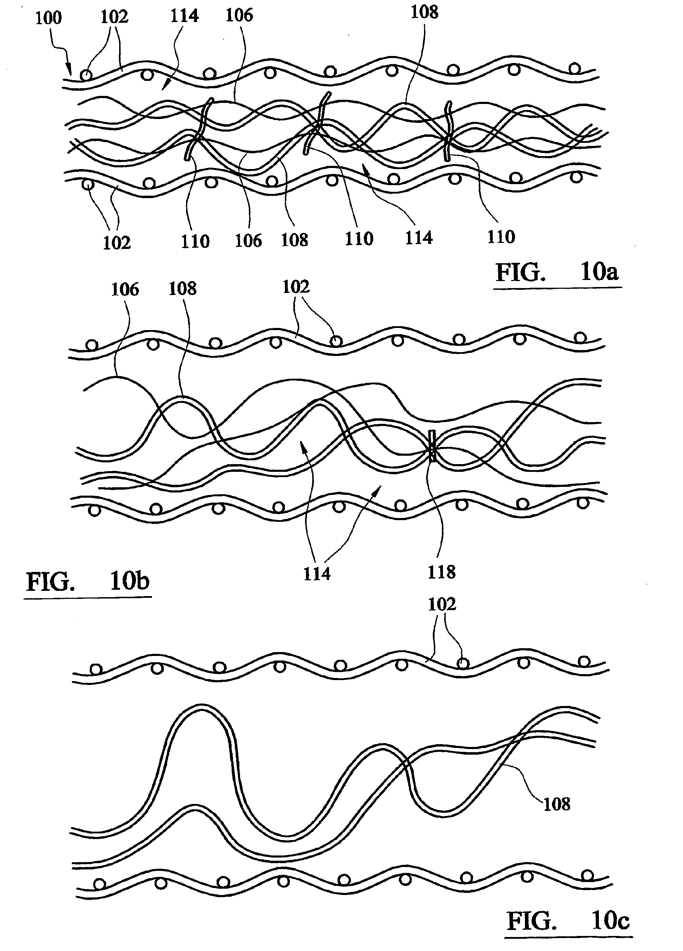

At the time of deployment, Figure 10a, the bag 100 is formed of a number of fibres 102 woven together to provided the necessary structure for containing the filling 104. The filling 104 itself is provided in the form of a series of wavy fibres of a first size 106 and second size 108, together with spacing fibres 110 which assist in maintaining the open position of the first size 106 and second size 108 fibres under compression. The result is an open structure with substantial gaps in the bag 100 to allow fluid communication through the bag 100 and substantial voids 114 between the fibres 106, 108, 110.

Six months or so after deployment, Figure 10b, the position has changed. Substantial amounts of tissue ingrowth has occurred. The tissue ingrowth serves in effect to provide nucleus material which resists compression of the nucleus and filling 104. The spacing fibres 110 are no longer required, therefore, having served their function of resisting compression of the fibres 106, 108 during the early days of the implant.

By providing the spacing fibres 110 from a bio-absorbable material which is relatively quickly absorbed, within 6 months or so, the spacing fibres 110 are removed from the equation. The tissue they served as a scaffold for usefully remains, but the fibres 110 themselves have gone in most places. A few remains 118 of such fibres 110 may remain. As a result of these fibres 110 going, there is no restriction on the expansion of the voids 114 by the spacing between fibres 106, 108 increasing. The tissue growth itself provides the expansive pressure for this to happen.

The non-bioabsorbable fibres 102 of the bag 100 remain, as do the fibres 106, 108 to provide assistance to the overall structure.

Figure 10c shows the position some 2 years or so after deployment. Yet further tissue growth has occurred and the regenerated tissue now provides the majority of the nucleus function. With this mainly biological provision of the necessary structure, there is less need for the fibres 106, 108. As the fibres 106 are also provided from a bio-absorbable material, these too are disappearing. Different time periods for bip-absorption to occurred are possible through selection of the material used. The removal of the fibres 106 allows the remaining fibres 108 to expand still further.

So as to accurately gauge the size of bag required and amount of filling needed, it is possible to measure the inflated volume of an inflatable bag inserted into the space vacated by the removed nucleus material.

As described in the previous embodiments, the implant is generally in the form of a bag and filling. However, in certain case it may be possible to use the filling without a bag. This is particularly the case where the removal of the disc material to form the void which needs filling is well defined, for instance due to its being bounded by the natural annulus and/or sound nucleus material.

In such cases, the benefits according to the present invention are still provided due to the different approach to the replacement of the nucleus material taken through its replacement by fibres. Again the present invention aims to provide a phased transition from a solution based on a non-biological mechanism to a combination of biological and non-biological mechanisms and potentially even on to a predominantly or even exclusively biological mechanism.

Fibres of the types, materials and configurations described above can be used in this embodiment. Once again, when exposed to such alien materials the body's reaction is to try and isolate the material by providing tissue growth around it.

## Claims

1. A spinal implant, the implant including a porous component (7) and one or more filling elements (22) provided within the porous component (7), in which one or more materials used in the porous component (7) is bio-absorbable,
**characterized in that** the bio-absorbable material restrains the porous component (7) in a first state, the bio-absorption of the material allowing the porous component (7) to assume a second state.

2. The implant of claim 1 in which the implant is a partial nucleus pulposus replacement or a total nucleus replacement.

3. The implant of claim 1 or claim 2 in which the porous component (7) is a bag or other form of container having an opening to permit the insertion of the one or more filling elements (22).

4. The implant of any preceding claim in which the porous component (7) is made of fabric, particularly a woven fabric.

5. The implant of any preceding claim in which the pores in the porous component (7) have at least one cross-sectional dimension that is less than the smallest cross-sectional dimension of the filling elements.

6. The implant of any preceding claim in which the porous component (7) is configured and/or formed of and/ or provided with one or more materials to promote tissue growth, particularly tissue ingrowth through the porous component (7) and/or between the porous component (7) and one or more of the filling elements (22) and/or between two or more of the filling elements (22).

7. The implant of any preceding claim in which the one or more filling elements (22) is fibrous.

8. The implant of any preceding claim in which one or more filling elements (22) that are porous and/or define voids within themselves and/or between parts of a filling element (22) are provided.

9. The implant of any preceding claim in which one or more filling elements (22) are formed of unconstrained fibres and/or unbraided fibres and/or interlaced fibres.

10. The implant of any preceding claim in which one or more filling elements (22) are provided with aligned fibres.

11. The implant of any preceding claim in which one or more filling elements (22) are provided with wavy and/or curved and/or zig zag fibres.

12. The implant of any preceding claim in which one or more filling elements (22) with fibres which act to space each other from one another are provided.

13. The implant of any preceding claim in which one or more filling elements (22) with fibres of two or more different cross sections are provided.

14. The implant of any preceding claim in which one or more filling elements (22) with fibres provided in a first direction are provided, with one or more restraining fibres or material which surround and / or enclose and / or be wrapped around and / or contact a plurality of fibres.

15. The implant of any preceding claim in which one or more filling elements (22) are provided with peripheral fibres or material provided around the filling element, the peripheral fibres or material being wrapped around the filling elements in a spiral manner and / or criss-cross manner.

16. The implant of any preceding claim in which one or more filling elements (22) are provided with pieces provided therein, the pieces being intermixed with one or more fibres.

17. The implant of claim 16 in which the pieces are spheres, beads, blocks or the like

18. The implant of claim 8 in which the pores and/or voids and/or apertures ad/or gaps occurring in the filling elements (22) and/or there between are due to the manner of manufacture of the material from which it is formed or are supplemented with further pores and/or voids and/or apertures or gaps.

19. The implant of any preceding claim in which the one or more filling elements (22) are configured and/or formed of one or more materials to promote tissue growth, particularly tissue ingrowth through one or more filling elements (22) and/or between the porous component (7) and one or more filling elements (22) and/or between two or more of filling elements (22).

20. The implant of any preceding claim in which the bio-absorbable material restrains one or more of the filling elements (22), or a part thereof, in a first state, the bio-absorption of the material allowing one or more filling elements (22), or a part thereof, to assume a second state, the second state providing a greater internal volume for one or more filling elements (22) and/or greater porosity for one or more filling elements (22) and/or reduction in mass of one or more filling elements (22) and/or provide mare space for tissue ingrowth

## Patentansprüche

1. Wirbelsäulenimplantat, bei dem das Implantat eine poröse Komponente (7) und ein oder mehr Füllelemente (22), die in der porösen Komponente (7) vorgesehen sind, aufweist, wobei ein oder mehr in der porösen Komponente (7) verwendete Materialien bioresorbierbar sind,
**dadurch gekennzeichnet, dass** das bioresorbierbare Material die poröse Komponente (7) in einem ersten Zustand hält, wobei es die Bioresorption des Materials gestattet, dass die poröse Komponente (7) einen zweiten Zustand annehmen kann.

2. Implantat nach Anspruch 1, bei dem das Implantat als Teilersatz des Nucleus pulposus oder als Nukleus-Totalersatz dient.

3. Implantat nach Anspruch 1 oder 2, bei dem die poröse Komponente (7) ein Beutel oder eine andere Form von Behälter mit einer Öffnung ist, durch welche die ein oder mehr Füllelemente (22) eingeführt werden können.

4. Implantat nach einem der vorhergehenden Ansprüche, bei dem die poröse Komponente (7) aus Stoff, insbesondere aus einem Gewebe, besteht.

5. Implantat nach einem der vorhergehenden Ansprüche, bei dem die Poren in der porösen Komponente (7) zumindest eine Querschnittsabmessung aufweisen, die kleiner ist als die kleinste Querschnittsabmessung der Füllelemente.

6. Implantat nach einem der vorhergehenden Ansprüche, bei dem die poröse Komponente (7) zur Förderung des Gewebewachstums, insbesondere des Einwachsens von Gewebe durch die poröse Komponente (7) und/oder zwischen der porösen Komponente (7) und ein oder mehr Füllelementen (22) und/oder zwischen zwei oder mehr Füllelementen (22), mit ein oder mehr Materialien konfiguriert und/oder geformt und/oder versehen ist.

7. Implantat nach einem der vorhergehenden Ansprüche, bei dem die ein oder mehr Füllelemente (22) faserig sind.

8. Implantat nach einem der vorhergehenden Ansprüche, bei dem ein oder mehr Füllelemente (22) vorgesehen sind, die porös sind und/oder Lücken in sich selbst und/oder zwischen Teilen eines Füllelements (22) definieren.

9. Implantat nach einem der vorhergehenden Ansprüche, bei dem ein oder mehr Füllelemente (22) aus lockeren Fasern und/oder ungeflochtenen Fasern und/oder verflochtenen Fasern geformt sind.

10. Implantat nach einem der vorhergehenden Ansprüche, bei dem ein oder mehr Füllelemente (22) mit ausgerichteten Fasern versehen sind.

11. Implantat nach einem der vorhergehenden Ansprüche, bei dem ein oder mehr Füllelemente (22) mit wellenförmigen und/oder gebogenen und/oder zickzackförmigen Fasern versehen sind.

12. Implantat nach einem der vorhergehenden Ansprüche, bei dem ein oder mehr Füllelemente (22) mit Fasern, die sich jeweils voneinander im Abstand halten, vorgesehen sind.

13. Implantat nach einem der vorhergehenden Ansprüche, bei dem ein oder mehr Füllelemente (22) mit Fasern mit zwei oder mehr verschiedenen Querschnitten vorgesehen sind.

14. Implantat nach einem der vorhergehenden Ansprüche, bei dem ein oder mehr Füllelemente (22) mit in einer ersten Richtung vorgesehenen Fasern vorgesehen sind, mit ein oder mehr Haltefasern oder Materialien, welche eine Vielzahl von Fasern umgeben und/oder diese einschließen und/oder um diese gewickelt sind und/oder mit diesen in Berührung stehen.

15. Implantat nach einem der vorhergehenden Ansprüche, bei dem ein oder mehr Füllelemente (22) mit um das Füllelement vorgesehenen peripheren Fasern oder Materialien vorgesehen sind, wobei die peripheren Fasern oder Materialien spiralförmig und/oder gitterartig um die Füllelemente gewickelt sind.

16. Implantat nach einem der vorhergehenden Ansprüche, bei dem ein oder mehr Füllelemente (22) mit darin vorgesehenen Stücken vorgesehen sind, wobei die Stücke mit ein oder mehr Fasern vermischt sind.

17. Implantat nach Anspruch 16, bei dem die Stücke Kugeln, Perlen, Blöcke oder dergleichen sind.

18. Implantat nach Anspruch 8, bei dem die Poren und/oder Lücken und/oder Öffnungen und/oder Zwischenräume, die in und/oder zwischen den Füllelementen (22) vorkommen, auf die Herstellungsart des Materials, aus dem es geformt ist, zurückzuführen sind oder mit weiteren Poren und/oder Lücken und/oder Öffnungen oder Zwischenräumen ergänzt sind.

19. Implantat nach einem der vorhergehenden Ansprüche, bei dem ein oder mehr Füllelemente (22) zur Förderung des Gewebewachstums, insbesondere des Einwachsens von Gewebe durch ein oder mehr Füllelemente (22) und/oder zwischen der porösen Komponente (7) und ein oder mehr Füllelementen (22) und/oder zwischen zwei oder mehr Füllelementen (22), mit ein oder mehr Materialien konfiguriert und/oder geformt sind.

20. Implantat nach einem der vorhergehenden Ansprüche, bei dem das bioresorbierbare Material ein oder mehr Füllelemente (22) oder einen Teil davon in einem ersten Zustand hält, wobei es die Bioresorption des Materials gestattet, dass ein oder mehr Füllelemente (22) oder ein Teil davon einen zweiten Zustand annehmen, wobei der zweite Zustand für ein größeres Innenvolumen für ein oder mehr Füllelemente (22) und/oder für eine größere Porosität für ein oder mehr Füllelemente (22) und/oder für eine Reduzierung der Masse von ein oder mehr Füllelementen (22) sorgt und/oder mehr Raum zum Einwachsen von Gewebe bietet.

## Revendications

1. Implant vertébral, l'implant comprenant un composant poreux (7) et un ou plusieurs éléments de remplissage (22) fournis à l'intérieur du composant poreux (7), dans lequel une ou plusieurs matières utilisées dans le composant poreux (7) sont bioabsorbables,
**caractérisé en ce que** la matière bioabsorbable retient le composant poreux (7) dans un premier état, la bioabsorption de la matière permettant au composant poreux (7) de prendre un deuxième état.

2. Implant selon la revendication 1 dans lequel l'implant est un remplacement partiel du noyau gélatineux ou un remplacement complet du noyau.

3. Implant selon la revendication 1 ou la revendication 2 dans lequel le composant poreux (7) est un sac ou une autre forme de contenant présentant une ouverture pour permettre l'introduction dudit un ou plusieurs éléments de remplissage (22).

4. Implant selon l'une quelconque des revendications précédentes dans lequel le composant poreux (7) est fabriqué en tissu, particulièrement un tissu tissé.

5. Implant selon l'une quelconque des revendications précédentes dans lequel les pores du composant poreux (7) ont au moins une dimension en coupe transversale qui est inférieure à la plus petite dimension en coupe transversale des éléments de remplissage.

6. Implant selon l'une quelconque des revendications dans lequel le composant poreux (7) est configuré et/ou formé et/ou pourvu d'une ou plusieurs matières pour favoriser une croissance tissulaire, particulièrement une interposition tissulaire à travers le composant poreux (7) et/ou entre le composant poreux (7) et un ou plusieurs des éléments de remplissage (22) et/ou entre deux éléments de remplissage (22) ou plus.

7. Implant selon l'une quelconque des revendications précédentes dans lequel ledit un ou plusieurs éléments de remplissage (22) est fibreux.

8. Implant selon l'une quelconque des revendications précédentes dans lequel un ou plusieurs éléments de remplissage (22) qui sont poreux et/ou définissent des vides à l'intérieur d'eux-mêmes et/ou entre des parties d'un élément de remplissage (22) sont fournis.

9. Implant selon l'une quelconque des revendications précédentes dans lequel un ou plusieurs éléments de remplissage (22) sont formés de fibres non contraintes et/ou fibres non tressées et/ou fibres entrelacées.

10. Implant selon l'une quelconque des revendications précédentes dans lequel un ou plusieurs éléments de remplissage (22) sont pourvus de fibres alignées.

11. Implant selon l'une quelconque des revendications précédentes dans lequel un ou plusieurs éléments de remplissage (22) sont pourvus de fibres ondulées et/ou courbes et/ou en zigzag.

12. Implant selon l'une quelconque des revendications précédentes dans lequel un ou plusieurs éléments de remplissage (22) sont fournis avec des fibres servant à les espacer les uns des autres.

13. Implant selon l'une quelconque des revendications précédentes dans lequel un ou plusieurs éléments de remplissage (22) sont fournis avec des fibres de deux ou plusieurs sections transversales différentes.

14. Implant selon l'une quelconque des revendications précédentes dans lequel un ou plusieurs éléments de remplissage (22) sont fournis avec des fibres agencées dans une première direction, avec une ou plusieurs fibres ou matière de retenue qui entourent et/ou enferment et/ou enveloppent complètement et/ou touchent une pluralité de fibres.

15. Implant selon l'une quelconque des revendications précédentes dans lequel un ou plusieurs éléments de remplissage (22) sont fournis avec des fibres ou une matière périphérique situées autour de l'élément de remplissage, les fibres ou la matière périphérique étant enroulées autour des éléments de remplissage en spirale et/ou d'une manière entrecroisée.

16. Implant selon l'une quelconque des revendications précédentes dans lequel un ou plusieurs éléments de remplissage (22) sont fournis avec des pièces situées à l'intérieur, les pièces étant entremêlées avec une ou plusieurs fibres.

17. Implant selon la revendication 16 dans lequel les pièces sont des sphères, des billes, des blocs ou des matières similaires.

18. Implant selon la revendication 8 dans lequel les pores et/ou les vides et/ou les ouvertures et/ou les interstices présents dans les éléments de remplissage (22) et/ou entre ceux-ci résultent du procédé de fabrication utilisé pour la matière dans laquelle ils sont réalisés ou complétés par d'autres pores et/ou vides et/ou ouvertures ou interstices.

19. Implant selon l'une quelconque des revendications précédentes dans lequel ledit un ou plusieurs éléments de remplissage (22) est configuré et/ou réalisé dans une ou plusieurs matières pour favoriser une croissance tissulaire, particulièrement une interposition tissulaire à travers un ou plusieurs des éléments de remplissage (22) et/ou entre le composant poreux (7) et un ou plusieurs éléments de remplissage (22) et/ou entre deux éléments de remplissage (22) ou plus.

20. Implant selon l'une quelconque des revendications précédentes dans lequel la matière bioabsorbable retient un ou plusieurs desdits éléments de remplissage (22), ou une partie de ceux-ci, dans un premier état, la bioabsorption de la matière permettant à un ou plusieurs éléments de remplissage (22), ou une partie de ceux-ci, de prendre un deuxième état, le deuxième état fournissant un volume interne plus grand pour un ou ou plusieurs éléments de remplissage (22) et/ou une plus grande porosité pour un ou plusieurs éléments de remplissage (22) et/ou une réduction de la masse d'un ou plusieurs éléments de remplissage (22) et/ou de fournir plus d'espace pour une interposition tissulaire.
